# EUROPEAN PATENT SPECIFICATION

(11) **EP 2 022 446 B1**
(45) Date of publication and mention of the grant of the patent: **04.04.2012**
(21) Application number: 07113408.4
(22) Date of filing: 30.07.2007
(51) Int. Cl.: A61F 2/00, A61K 6/00, A61L 24/00, A61L 27/46, A61L 27/58

(54) **Bio-degenerable bone cement and manufacturing method thereof**
Biologisch abbaubarer Knochenzement und Herstellungsverfahren dafür
Ciment d'os biodégradable et son procédé de fabrication

(43) Date of publication of application: 11.02.2009
(73) Proprietor: Purzer Pharmaceutical Co., Ltd., Taipei City 105 (TW)
(72) Inventor: Lin, Feng-Huei, Taipei 100 (TW); Kuo, Tzong-Fu, Taipei 106 (TW); Wu, Chang-Chin, Taipei 100 (TW); Lin, Min-Huei, Taipei 100 (TW); Yang, Kai-Chiang, Taipei 100 (TW)
(74) Representative: Viering, Jentschura & Partner

(56) References cited:
- WO-A-03/002490
- US-A- 4 612 053
- US-A- 6 124 373
- US-A- 6 153 664
- HAKIMIMEHR ET AL: "In-situ preparation of poly(propylene fumarate)-hydroxyapatite composite" BIOMATERIALS, ELSEVIER SCIENCE PUBLISHERS BV., BARKING, GB, vol. 26, no. 35, December 2005 (2005-12), pages 7297-7303, XP005027832 ISSN: 0142-9612
- ALBERT K. SHUNG ET AL.: "kinetics of poly(propylene fumarate) synthesis by step polymerisation of diethyl fumarate and propylene glycol using zinc chloride as a catalyst" J. BIOMAT. SCI. POLYMER, vol. 13, no. 1, 2002, pages 95-108, XP008085841

## Description

### FIELD OF THE INVENTION

The present invention relates to a bio-degenerable bone cement and a manufacturing method thereof, and more particularly to a diphasic material composed of poly (propylene fumarate) (PPF) and tetracalcium phosphate (Ca₄O(PO)₂ or TTCP)/anhydrous dicalcium phosphate (CaHPO₄ or DCPA) which is used as a bone cement material for filling damaged bones and its related manufacturing method.

### BACKGROUND OF THE INVENTION

As our living standard improves and medical treatment advances, we can foresee an ageing population accompanied with all kinds of illnesses such as osteoporosis and its related complications, and osteoporosis becomes a serious problem to the health of the elderly people. The most common complication of osteoporosis is the vertebral compression fracture, since osteoporosis reduces the bone mineral density and makes our bone fragile. In the United States, approximately 700,000 people are suffering vertebral compression fracture caused by osteoporosis each year, and approximately 100,000 of these patients require hospitalization.

In recent years, medical professionals start applying vertebroplasty in the treatment of vertebral compression fracture. The principle of vertebroplasty is to inject bone cements into the position of a bone fracture to secure the bone and achieve the effect of releasing the pain.

At present, most bone cements applied for vertebroplasty are primarily made of polymethyl methacrylate (PMMA), because the PMMA bone cement can provide sufficient strength for the bone fracture at an early stage. However, the PMMA bone cement still has the following drawbacks to be overcome:
1. The high temperature of the polymerization reaction may burn the patient's sensory nerve ending.
2. The remained methyl methacrylate (MMA) liquid is a toxic matter, and any leak may cause vein incompetence and result in pulmonary embolism.
3. Compared with the natural bone structure, PMMA bone cement comes with a too-large strength after PMMA is hardened. Stresses may be concentrated at a point, which may result in osteoporosis and bone fracture for the second time.
4. The PMMA bone cement is a material which is not bio-degenerable, and thus it may hinder the bone remolding process. If the bone cement leaks during a surgery, a second surgical operation may be required.
5. The PMMA bone cement cannot be bonded with the bone directly.

To overcome the aforementioned drawbacks, many manufacturers have attempted to add a certain material such as ceramic particle or demineralized bone matrix (DBM) to improve the property of the PMMA bone cement, so as to increase the biological activity and lower the too-large strength. However, the effect was not so great.

In addition to the PMMA bone cement, a recently popular bone cement is the ceramic series bone cement such as the calcium phosphate cement (CPC), and this type of bone cement has the following advantages over the PMMA bone cement:
1. The bio-compatibility of calcium phosphate cement (CPC) is very high, since its structure and the bone tissues are basically consisted of calcium phosphate, and thus the calcium phosphate cement (CPC) can be bonded with the bone directly.
2. Since the structure of the CPC is the same with our bone tissues, the cement can be used for bone remodeling directly without requiring a second surgical operation to remove the cement. With this property, some growth factors are added in the cement to step up the bone repair and remodeling.
3. The strength of the CPC is close to the strength of our bones, and thus the CPC will not crush other bones.

WO 03/002490 discloses a method for the manufacture of a biodegenerable bone graft, comprising mixing diethyl fumarate and propylene glycol in the presence of ZnCl₂ and hydroquinine to synthetize a poly(propylene fumarate) polymer. This european application does not mention bone cement nor combination of specific calcium phosphates : TTCP and DCPA.

US 6153664 discloses a method for the manufacture of a biodegenerable bone cement, comprising mixing calcium phosphate such as hydroxyapatite in a solution of PPF and N vinyl pyrrolidone.

US 6124373 discloses a method for the manufacture of a biodegenerable bone cement, comprising mixing fumaryl chloride and propylene glycol in the presence of benzoyl peroxide and anhydrous potassium carbonate to synthetize a poly(propylene fumarate) polymer. The resulting PPF polymer is then mix with fillers (TCP) in the presence of N-vinyl pyrrolidone.

However, the aforementioned calcium phosphate cement (CPC) comes with an insufficient strength at an early stage, and it cannot meet clinical requirements. Ob viously, it is necessary to overcome the shortcomings of the prior art bone cement and develop a new bone cement to enhance the effect of vertebroplasty for the treatment of vertebral fractures.

### SUMMARY OF THE INVENTION

In view of the foregoing shortcomings of the prior art, the inventor of the present invention based on years of experience in the related industry to conduct extensive researches and experiments, and finally developed a bio-degenerable bone cement and a manufacturing method thereof in accordance with the present invention to overcome the shortcomings of the

### prior art.

The primary objective of the present invention is to provide a bio-degenerable bone cement and a manufacturing method thereof, such that the manufactured poly (propylene fumarate) (PPF) is mixed and dissolved uniformly in N-vinylpyrrolidone (N-VP), and tetracalcium phosphate (Ca40(PO)2 or TTCP)/anhydrous dicalcium phosphate (CaHPO₄ or DCPA) is dissolved in the N-VP/PPF solution, and a baking powder (BP) is dissolved in the solution, and the baking powder and the solution are mixed uniformly to allow a complete solidification in room temperature and produce a bio-degenerable bone cement in accordance with the present invention. The bone cement complies with the patent application requirements and comes with many advantages. For instance, the bone cement can be injected into the position of a bone fracture, and it is bio-degenerable, and comes with better mechanical properties. In addition, the temperature of polymerization is lower than that of the prior art PMMA bone cement, and the pressure resistance is closer to our bone than that of the prior art PMMA bone cement PMMA. The bone cement has a high bio-compatibility and an impenetrability of radiation, and thus such bone cement provides a great application to the vertebroplasty.

### BRIEF DESCRIPTION OF THE DRAWING

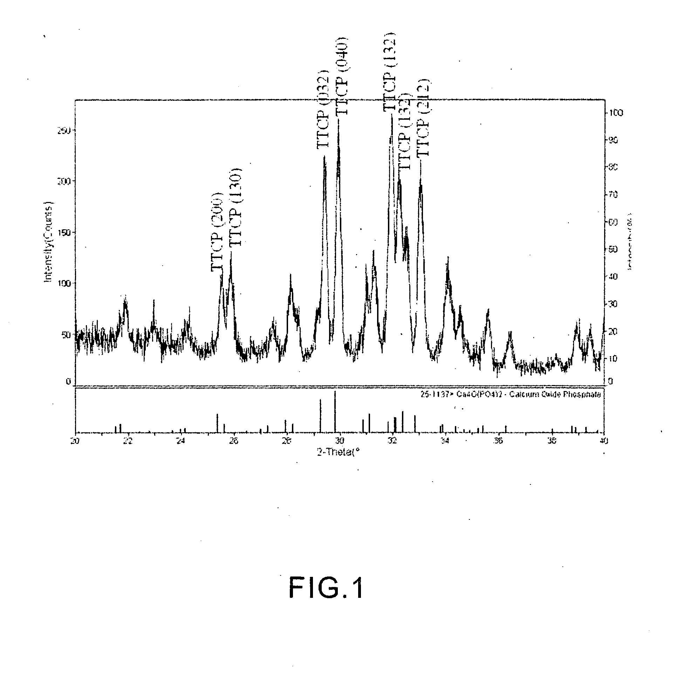

FIG. 1 shows the comparison of an qualitative analysis of the tetra-calcium phosphate (TTCP) measured by a X-ray diffractor with a standard spectrum of the JCPDS database in accordance with the present invention;
FIG. 2 shows the comparison of an qualitative analysis of a finished good of calcium-deficient hydroxyapatite (dHAP) made of the tetra-calcium phosphate (TTCP)/anhydrous dicalcium phosphate (CaHPO₄ or DCPA) and measured by a X-ray diffractor with a standard spectrum of the JCPDS database in accordance with the present invention; and
FIG. 3 shows a photo of the material surface of a bone cement taken through an electronic microscope in accordance with the present invention.

Related chemical equations of the present invention are listed below:
TTCP Synthesis Equation:

   Ca₂P₂O₇ + 2CaCO₃ → Ca₄(PO₄)₂O + 2CO₂
CPC Hydration Equations:
Reaction at an Early Stage:

   2Ca₄(PO₄)₂O + 2CaHPO₄ + H₂O → Ca₁₀(PO₄)(OH)₂
Reaction at a Later Stage:

   2Ca₄(PO₄)₂O + 2CaHPO₄ + H₂O → Ca_{10-X}(HPO₄)_{X}(PO₄)_{6-X}(OH)_{2-X},

   where 0≦X≦1.

### DETAILED DESCRIPTION OF THE PREFERRED EMBODIMENTS

To make it easier for our examiner to understand the objective of the invention, its structure, innovative features, and performance, we use a preferred embodiment together with the attached drawings for the detailed description of the invention.

The present invention relates to a bio-degenerable bone cement and a manufacturing method thereof, and the bio-degenerable bone cement uses a mixture of poly (propylene fumarate) (PPF) and a diphasic material of tetracalcium phosphate (Ca₄O(PO)₂ or TTCP)/anhydrous dicalcium phosphate (CaHPO₄) or anhydrous dicalcium phosphate (CaHPO₄ or DCPA) to obtain a bio-degenerable bone cement. Since poly (propylene fumarate) (PPF) comes bio-degenerable bone cement. Since poly (propylene fumarate) (PPF) comes with a low polymerization temperature and a bio-degenerable feature, such bone cement can be used as an injective bone cement, and the tetra-calcium phosphate (TTCP)/anhydrous dicalcium phosphate (CaHPO₄ or DCPA) form a porous structure after the solidification, and the structure is very similar to our bone structure. In addition, the bone cement has a relatively high bio-compatibility, and thus it can be used directly for bone remodeling and rebuilding. The present invention mixes the aforementioned two materials to be filled in human bones.

The method of preparing polymer PPF of the bone cement material in accordance with a preferred embodiment of the present invention is described as follows:

The method includes two steps, wherein diethyl fumarate (DEF) and propylene glycol (PG) are mixed as the primary raw materials, and zinc chloride (ZnCl₂) is added to serve as a catalyst and hydroquinone (Hq) is added to serve as a crosslink breaker for producing the required polymer PPF.

In the first step, diethyl fumarate (DEF), propylene glycol (PG), zinc chloride (ZnCl₂) and hydroquinone (Hq) in a molar proportion of 1: 3: 0.01: 0.002 are mixed uniformly to increase the temperature up to 100°C, and then the mixture is heated to 150°C. Since it is necessary to maintain an air insulating status during the reaction process, nitrogen gas is used and passed through during the reaction process. In the process, diethyl fumarate (DEF) and propylene glycol (PG) are reacted to form ethanol, and the ethanol is condensed by a condensation pipe. If no more ethanol is condensed, it shows that the first step of the reaction is completed.

In the second step, the temperature is set at 100°C, and the pressure is reduced to 0.1 torr. In this process, the unreacted propylene glycol (PG) will be condensed and separated, and then the temperature is increased to 130-150°C in the next coming two hours. Now, the reaction is started to form a polymer PPF. Within two hours, the temperature is increased to 200°C, and then the constant temperature at 200°C is maintained for 12 hours before the mixture is cooled to room temperature, so as to produce a viscous liquid in amber color, and this liquid is the required product of polymer PPF.

Since the polymer PPF still contains the catalyst (zinc chloride) and the crosslink breaker (hydroquinone), therefore it is necessary to purify and remove the catalyst and crosslink breaker. The purification procedure comprises the steps of: dissolving the polymer PPF into dichloromethane with a volume ratio of 1 : 1; adding hydrogen chloride (HCl) with a IN concentration to remove the catalyst (zinc chloride); using the same volume of secondary water and saltwater for repeated extractions to remove the organic solvent (dichloromethane); adding a concentrate sulfuric acid to remove the extra water moisture; and adding cold ethyl ether into the remaining polymer PPF and dichloromethane to remove the extra crosslink breaker (hydroquinone). After this procedure, most of the polymer PPFs are purified. However, dichloromethane is toxic, and thus it is necessary to vacuum and dry the finished goods to remove the extra organic solvent. The purified polymer PPF should be stored at a temperature below -20°C when it is not in use.

The method of preparing tetra-calcium phosphate (TTCP)/anhydrous dicalcium phosphate (CaHPO₄ or DCPA) in the bone cement material in accordance with a preferred embodiment of the present invention is described as follows:

In the first step, one mole of calcium pyrophosphate (Ca₂P₂O₇) powder and two moles of calcium carbonate (CaCO₃) powder are mixed thoroughly and uniformly, and then the mixture is laid flatly on a platinum crucible, and the platinum crucible containing the mixture is put into a silicon carbon (SiC) high temperature furnace for a high temperature sintering.

In the second step, the powder mixture is heated with a heating rate of 10°C/min to a sintering temperature of 1440°C, and such temperature is maintained for three hours, and then the powder mixture is quenched rapidly to room temperature to obtain tetra-calcium phosphate (TTCP). In this embodiment, a mortar grinder is used for grinding TTCP into powder, and then the powder is sieved and filtered (by a sieve model no. Mesh No. 106) and the equation of the reaction is given below:

Ca₂P₂O₇ + 2CaCO₃ → Ca₄(PO₄)₂O + 2CO₂

In the third step, the tetra-calcium phosphate (TTCP) powder obtained from the above procedure is mixed uniformly with anhydrous dicalcium phosphate (CaHPO₄ or DCPA) in a molar ratio of 1: 1 to obtain the tetra-calcium phosphate (TTCP)/anhydrous dicalcium phosphate (CaHPO₄ or DCPA) diphasic bone cement.

In addition, the solidification mechanism of the tetra-calcium phosphate (TTCP)/anhydrous dicalcium phosphate (CaHPO₄ or DCPA) diphasic cement is described as follows. Since tetra-calcium phosphate (TTCP) and anhydrous dicalcium phosphate (CaHPO₄ or DCPA) react with water easily, therefore hydroxyapatite will be formed at an early stage of hydration, and then calcium-deficient hydroxyapatite (dHAP) will be formed later, and such calcium-deficient hydroxyapatite (dHAP) will provide a needle-like structure that forms crystals thereon by using HAP as a crystal nucleus, and the interlaced crystals constitute a stable structure to achieve the required solidification result, and the equations of the related reactions are given below:
Reaction at an Early Stage:

   2Ca₄(PO₄)₂O + 2CaHPO₄ + H₂O → Ca₁₀(PO₄)(OH)₂
Reaction at a Later Stage:

   2Ca₄(PO₄)₂O + 2CaHPO₄ + H₂O → Ca_{10-X}(HPO₄)_{X}(PO₄)_{6-X}(OH)_{2-X},

   where 0≦X≦1

Referring to FIG, 1 for a comparison of an qualitative analysis of the tetra-calcium phosphate (TTCP) measured by a X-ray diffractor with a standard spectrum of the JCPDS database in accordance with the present invention, the peak of the prepared material matches with the peak of the standard spectrum. The necessary conditions of setting the X-ray diffraction includes: The interval between atomic layers must be equal to the wavelength of the radiation, and the scattering environment of the scattering center must have a high regularity, so that a specific peak of the diffraction of the material can be used for the qualitative analysis of the crystalline phase of the material.

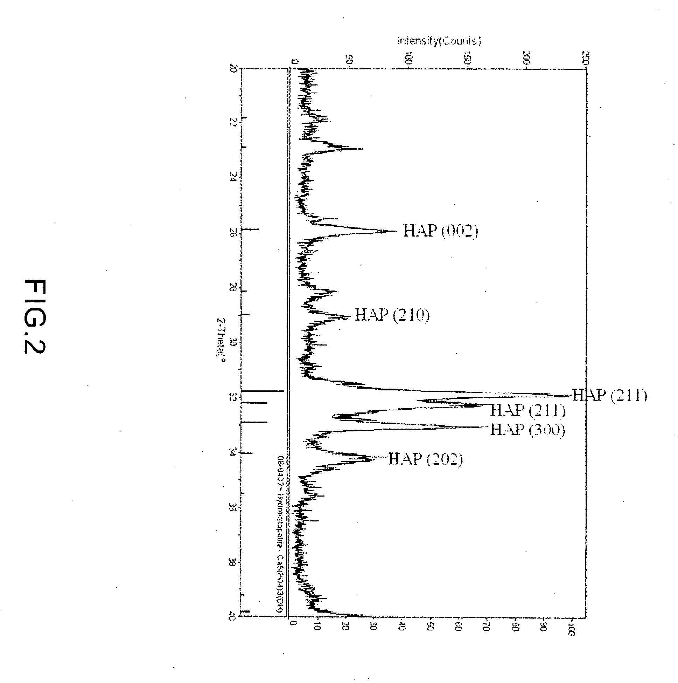

Referring to FIG. 2 for a comparison of an qualitative analysis of a finished good of calcium-deficient hydroxyapatite (dHAP) made of tetra-calcium phosphate (TTCP)/anhydrous dicalcium phosphate (CaHPO₄ or DCPA) and measured by a X-ray diffractor with a standard spectrum of the JCPDS database in accordance with the present invention, the finished good of the calcium-deficient hydroxyapatite (dHAP) after hydration is definitely similar to the composition of human mineralized bones

After the polymer PPF and the tetra-calcium phosphate (TTCP)/anhydrous dicalcium phosphate (CaHPO4 or DCPA) are prepared, mixing and preparation are carried out according to the following procedure:

Firstly, poly (propylene fumarate) (PPF) is dissolved and mixed uniformly in N-vinylpyrrolidone (N-VP).

Secondly, tetra-calcium phosphate (TTCP)/anhydrous dicalcium phosphate (CaHPO₄ or DCPA) is dissolved in the N-VP/PPF solution.

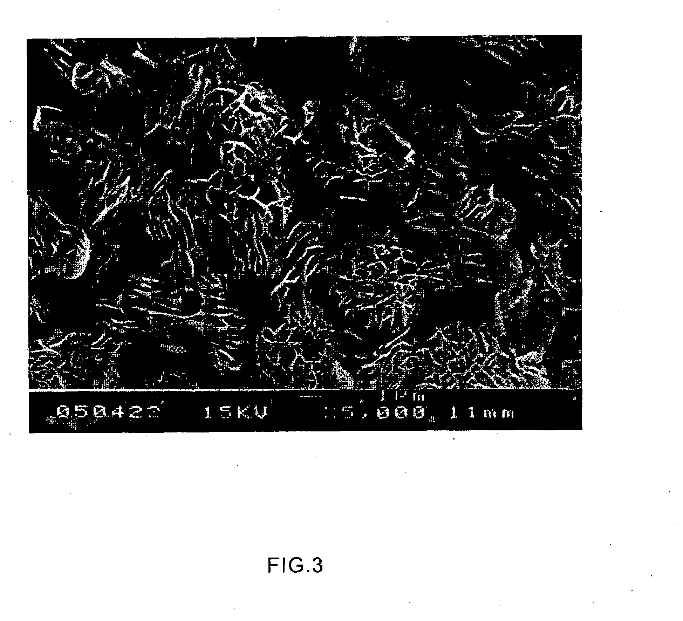

Finally, a baking powder (BP) is dissolved in the solution, and they are mixed uniformly and poured into a mold. The mixture is solidified completely at room temperature to produce the bio-degenerable bone cement in accordance with the present invention. Referring to FIG. 3 for a photo taken though an electronic microscope, the surface of the bone cement in accordance with the present invention is shown.

In summation of the description above, the bone cement made of poly (propylene fumarate) (PPF) and tetra-calcium phosphate (TTCP)/anhydrous dicalcium phosphate (CaHPO₄ or DCPA) in accordance with the present invention complies with the patent application requirements and includes the following advantages:
1. The bone cement can be injected into the position of a bone fracture.
2. The bone cement comes with a bio-degenerable feature and better mechanical properties.
3. The appropriate temperature of the bone cement is lower than that of the prior art bone cement PMMA.
4. The pressure resistance of the bone cement is closer to that of our bones than the prior art PMMA bone cement PMMA.
5. The bone cement comes with a high bio-compatibility.
6. The bone cement comes with an impenetrability of radiation, and thus it is not necessary to add a developer such as a barium sulfate developer for a better developing effect than the prior art PMMA bone cement).
7. The bone cement provides a great application to the vertebroplasty.

It is to be understood, however, that even though numerous characteristics and advantages of the present invention have been set forth in the foregoing description, together with details of the structure and function of the invention, the disclosure is illustrative only, and changes may be made in detail, especially in matters of shape, size, and arrangement of parts within the principles of the invention to the full extent indicated by the broad general meaning of the terms in which the appended claims are expressed.

## Claims

1. A bio-degenerable bone cement, which is obtained by
- mixing poly(propylene fumarate) and a calcium phosphate cement powder obtainable by mixing tetracalcium phosphate (Ca₄O(PO₄)₂) and anhydrous dicalcium phosphate (CaHPO₄); and
- cross-linking the mixture with N-vinylpyrrolidone, wherein the mixture is solidified at room temperature.

2. The bio-degenerable bone cement as recited in claim 1, wherein the tetracalcium phosphate is composed of calcium pyrophosphate (Ca₂P₂O₇) and calcium carbonate (CaCO₃).

## Patentansprüche

1. Biologisch zersetzbarer Knochenzement, der erzeugt wird durch:
- Vermischen von Poly-(Propylenfumaraten) und Calciumphosphat-Zementpulver, das erzeugbar durch Vermischen von Tetracalciumphosphat (Ca₄O(PO₄)₂) und wasserfreien Dicalciumphosphat (CaHPO₄) ist, und
- Vernetzen der Mischung mit N-Vinylpyrrolidonen,
wobei die Mischung bei Raumtemperatur verfestigt wird.

2. Biologisch zersetzbarer Knochenzement nach Anspruch 1, wobei das Tetracalciumphospat Calciumpyrophosphate (Ca₂P₂O₇) und Calciumcarbonate (CaCO₃) umfasst.

## Revendications

1. Ciment d'os biodégradable qui est obtenu par :
- mélange d'une poudre de ciment de fumarate de polypropylène et un phosphate de calcium qui peut être obtenue en mélangeant du tétracalcium phosphate (Ca₄O(PO₄)₂) et du phosphate dicalcique anhydre (CaHPO₄) et
- réticulation du mélange avec du N-vinylpyrrolidone,
le mélange étant solidifié à température ambiante.

2. Ciment d'os biodégradable selon la revendication 1 dans lequel le tétracalcium phosphate est composé de pyrophosphate de calcium (Ca₂P2O₇) et de carbonate de calcium (CaCO₃).
